# EUROPEAN PATENT SPECIFICATION

(11) **EP 3 011 926 B1**
(45) Date of publication and mention of the grant of the patent: **12.07.2017**
(21) Application number: 15191182.3
(22) Date of filing: 23.10.2015
(51) Int. Cl.: A61B 50/10, A47B 51/00, A47B 57/06

(54) **MEDICAL INSTRUMENT CABINET**
SCHRANK FÜR MEDIZINISCHE INSTRUMENTE
ARMOIRE D'INSTRUMENTS MÉDICAUX

(30) Priority: 23.10.2014 US 201462067490 P; 04.09.2015 US 201514845456
(43) Date of publication of application: 27.04.2016
(73) Proprietor: Stanley Convergent Security Solutions, Inc., Naperville IL 60563 (US)
(72) Inventor: Bjorum, Justin, M., Wyoming, MI 49418 (US); McCarthy, Patrick, G., Grand Rapid, MI (US)
(74) Representative: SBD IPAdmin

(56) References cited:
- WO-A2-98/25569
- CN-U- 203 291 040
- CN-U- 203 852 437
- US-B1- 6 755 492

## Description

This invention relates generally to cabinets for storing medical instruments.

Medical instruments, such as endoscopes, are typically stored in cabinets on stationary managers of various sizes. For example, the height of the cabinet is typically proportional to the length of the endoscope in order to ensure that the endoscopes can hang freely without touching any other endoscope within the cabinet or portion of the cabinet (other than the endoscope handle holder). As such, some endoscopes are very long and require very tall cabinets.

The current manufacturing trend is to make longer endoscopes in order to accommodate the increasing height and weight of the world population. Such longer endoscopes are more difficult to store and access from storage to the height of the cabinets increasing along with the scopes.

It is an object of the invention to provide a design that can store endoscopes of different lengths.

In accordance with the present invention, which is defined in independent claim 1 and the dependent claims 2 - 9, an improved medical instrument cabinet is employed. The cabinet may include a first side wall, a rear wall connected to the first side wall, and an adjustable support assembly disposed on the first side wall or the rear wall. The adjustable support assembly includes a housing, a slide assembly connected to the housing, a holder assembly movable relative to the housing and connected to the slide assembly, a belt connected to the holder assembly, and a counterweight connected to the belt.

The accompanying drawings illustrate preferred embodiments of the invention according to the practical application of the principles thereof, and in which:
FIG. 1 is a perspective view of a cabinet according to the invention;
FIG. 2 is a front perspective view of a sliding door;
FIG. 3 is a rear view of the sliding door of FIG. 2;
FIG. 4 is a front perspective view of the adjustable support assembly disposed inside the cabinet of FIG. 1; and
FIG. 5 is a partial rear perspective view of the adjustable support assembly of FIG. 4.

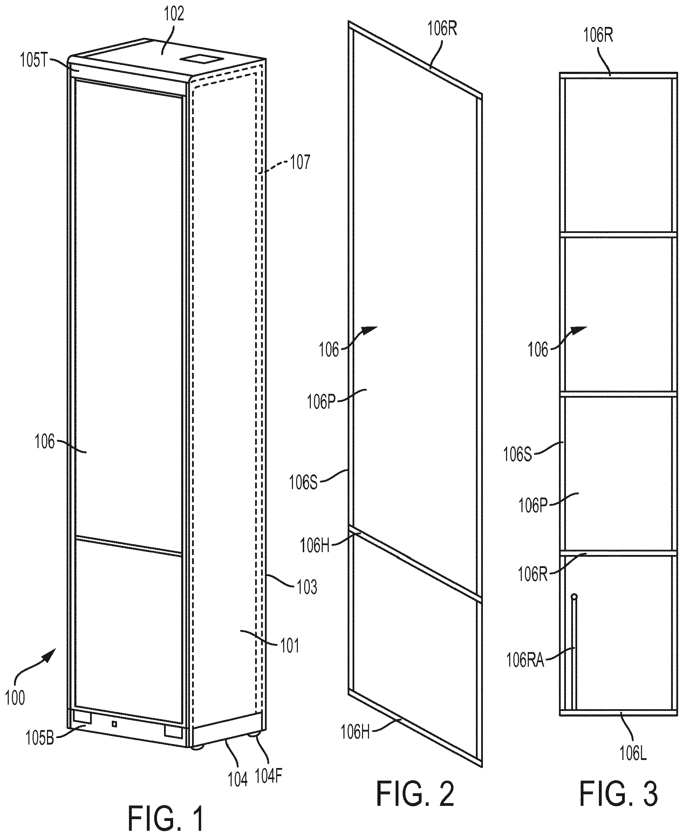

Referring to FIG. 1, a cabinet 100 for housing medical instruments, such as endoscopes, ultrasound probes or transesophageal echocardiogram (TEE) probes. Cabinet 100 preferably has two side walls 101, a rear wall 103, a top panel 102 and a bottom panel 104. Bottom panel 104 may include adjustable feet 104F attached thereto, as is well known in the art. Front panels 105T, 105B may be connected to the side walls 101, and top and bottom panels 102, 104, respectively, for maintaining the structural integrity of cabinet 100.

Referring to FIGS. 1-3, a sliding door 106 preferably covers the cabinet opening between front panels 105T, 105B. Preferably sliding door 106 comprises a flexible panel 106P attached and/or connected to glide strips 106S.

Sliding door 106 may also have handles 106H attached to panel 106P and/or glide strips 106S. Handles 106H may be made of extruded aluminum. Persons skilled in the art will recognize that handles 106H are preferably disposed on the front side of panel 106P.

Panel 106P may also have ribs 106R mounted thereon. Preferably ribs 106R are made of extruded aluminum. Persons skilled in the art will recognize that ribs 106R may be mounted on panel 106P via fasteners and/or adhesives.

Persons skilled in the art will recognize that sliding door 106 as described above preferably has no seams in panel 106P, facilitating cleaning thereof.

Cabinet 100 preferably has glide tracks 107 that engage glide strips 106S. With such construction, a user can lift sliding door 106 via handles 106H.

Panel 106P is preferably made of a thermoplastic material, such as ABS, or any other flexible membrane material. Glide strips 106S are preferably made of flexible materials, such as felt strips, which could be disposed on panel 106P. With such construction, both panel 106P and glide strips106S will follow along glide tracks 107. Therefore sliding door 106 will bend around the top of cabinet 100 and continue sliding downwardly along the rear of cabinet 100.

Persons skilled in the art shall recognize that sliding door 106 is preferably made of a single panel 106P. Having a single panel eliminates gaps between multiple panels, making cleaning thereof easier.

The bottom handle 106H and/or rib 106R may carry a rotatable handle 106RA. Such rotatable handle 106RA may be rotated about a substantially horizontal axis so that, as sliding door 106 is moved upwardly, the user can rotate rotatable handle 106RA downwardly to assist in the lifting and/or lowering operations of door 106.

The bottom handle 106H and/or rib 106R may carry a door latch 106L for latching or locking sliding door 106 to front panel 105B.

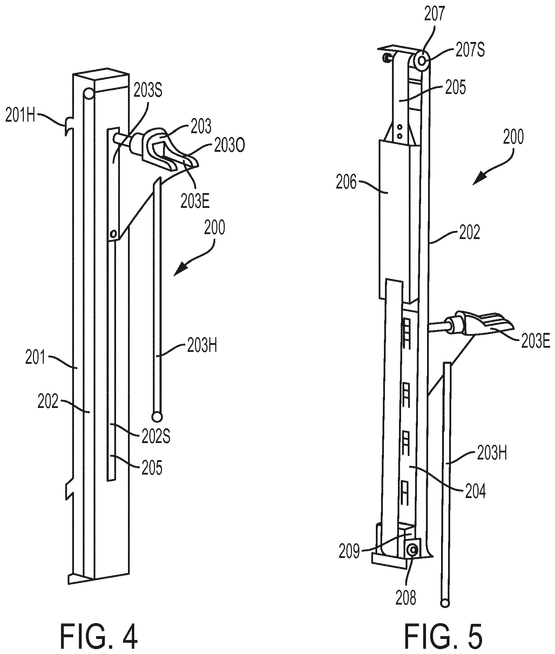

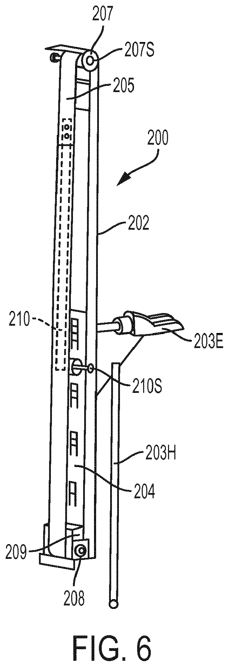

Referring to FIGS. 4-5, an adjustable support assembly 200 is disposed inside cabinet 100. Preferably support assembly 200 has a rear housing 201 with hooks 201 H that can latch into slots (not shown) provided on the inside of rear wall 103 and/or side walls 101. A front housing 202 is preferably attached to rear housing 201. Front housing 202 may have a slot 202S that allows the holder assembly 203 to move vertically therealong.

Holder assembly 203 preferably has an instrument holder 203E. Preferably, the portions of instrument holder 203E that contact the medical instrument are overmolded with an elastomeric material 2030.

Instrument holder 203E is attached to a plate 203S, which is in turn attached to a slide assembly 204. Slide assembly 204 is fully described in US Patent No. 7,802,856. Such slide assembly 204 is preferably an integrated ball bearing slide assembly. Slide assembly 204 is attached to front housing 202, and allows holder assembly 203 to move vertically. Such movement is further facilitated by attaching handle 203H to instrument holder 203E. Persons skilled in the art will recognize that slide assembly 204 will preferably have an integrated locking mechanism for maintaining holder assembly 203 in the lowest position as desired.

Instrument holder 203E and/or plate 203S may be attached to a belt 205 that moves along with instrument holder 203E and plate 203S when instrument holder 203E is moved vertically. As shown in FIG. 5, belt 205 extends vertically above instrument holder 203E, and wraps around an upper pulley 207 provided within rear and front housings 201, 202. Persons skilled in the art will recognize that pulley 207 is preferably disposed on a shaft 207S which is captured between rear and front housings 201, 202. Similarly, belt 205 extends vertically below instrument holder 203E, and wraps around a lower pulley 208 is provided within rear and front housings 201, 202. Persons skilled in the art will recognize that, because belt 205 is attached to plate 203S, belt 205 preferably covers slot 202S, preferably providing an easily cleanable surface.

Belt 205 is then preferably attached at both ends thereof to a counterweight 206. Persons skilled in the art shall recognize that counterweight 206 facilitates lifting instrument holder 203E. Furthermore, such counterweight 206 will also ensure a smooth downward motion when instrument holder 203E is moved to a lower position.

Persons skilled in the art will recognize that counterweight 206 may be replaced by a constant force spring 210 attached to belt 205. Constant force spring 210 may be disposed on a shaft 210S connected to front housing 202. Persons skilled in the art will recognize that using a constant force spring is advantageous because the constant force spring will exert a relatively consistent force regardless of the position of instrument holder 203 relative to front housing 202. Such consistent force preferably provides a counter-balance to the instrument holder 203 and medical instrument together.

A dampening block 209 may be provided above lower pulley 208. Preferably block 209 is made of soft rubber or foam. Persons skilled in the art shall recognize that block 209 preferably provides a cushioned stop for counterweight 206 when instrument holder 203E reaches its highest position.

Persons skilled in the art may recognize other additions or alternatives to the means disclosed herein. However, all these additions and/or alterations are considered to be equivalents of the present invention.

## Claims

1. A medical instrument cabinet (100) comprising:
a first side wall (101),
a rear wall (103) connected to the first side wall, and
an adjustable support assembly (200) disposed on the first side wall or the rear wall,
the adjustable support assembly comprising
a slide assembly (204),
**characterised in that** the adjustable support assembly further comprises:
a housing (201, 202) which is connected to the slide assembly,
a holder (203) movable relative to the housing and connected to the slide assembly,
a belt (205) connected to the holder assembly, and at least one of a counterweight (206) and a constant force spring (210) connected to the belt.

2. The medical instrument cabinet of Claim 1, wherein the holder assembly is at least partially covered with elastomeric material.

3. The medical instrument cabinet of Claim 1 or Claim 2, wherein the slide assembly is an integrated ball bearing slide assembly.

4. The medical instrument cabinet of any preceding claim, further comprising at least one of an upper pulley (207) and a lower pulley (208) disposed on the housing.

5. The medical instrument cabinet of Claim 4, wherein the belt contacts the at least one of the upper pulley and the lower pulley.

6. The medical instrument cabinet of any preceding claim, further comprising a glide strip (106S) disposed on the first side wall, and a sliding door (106) connected to the glide strip.

7. The medical instrument cabinet of Claim 6, wherein the sliding door comprises a flexible panel (106P) connected to the glide strip.

8. The medical instrument cabinet of Claim 6 or Claim 7, wherein the sliding door comprises handles (106H) attached to the flexible panel.

9. The medical instrument cabinet of any one of claims 6 to 8, wherein the sliding door does not have any seams.

## Patentansprüche

1. Schrank (100) für medizinische Instrumente, umfassend:
eine erste Seitenwand (101),
eine hintere Wand (103), die mit der ersten Seitenwand verbunden ist, und
eine einstellbare Stützanordnung (200), die an der ersten Seitenwand oder der hinteren Wand angeordnet ist, wobei die einstellbare Stützanordnung umfasst:
eine Schiebeanordnung (204),
**dadurch gekennzeichnet, dass** die einstellbare Stützanordnung ferner umfasst:
ein Gehäuse (201, 202), das mit der Schiebeanordnung verbunden ist,
einen Halter (203), der relativ zu dem Gehäuse bewegbar ist und mit der Schiebeanordnung verbunden ist,
einen Riemen (205), der mit der Halteranordnung verbunden ist, und
mindestens eines von einem Gegengewicht (206) und einer Feder (210) mit konstanter Kraft, die mit dem Riemen verbunden sind.

2. Schrank für medizinische Instrumente nach Anspruch 1, wobei die Halteranordnung mindestens teilweise mit elastomerem Material bedeckt ist.

3. Schrank für medizinische Instrumente nach Anspruch 1 oder Anspruch 2, wobei die Schiebeanordnung eine integrierte Kugellager-Schiebeanordnung ist.

4. Schrank für medizinische Instrumente nach einem der vorhergehenden Ansprüche, ferner umfassend mindestens eine von einer oberen Riemenscheibe (207) und einer unteren Riemenscheibe (208), die an dem Gehäuse angeordnet sind.

5. Schrank für medizinische Instrumente nach Anspruch 4, wobei der Riemen mit der mindestens einen von der oberen Riemenscheibe und der unteren Riemenscheibe in Kontakt steht.

6. Schrank für medizinische Instrumente nach einem der vorhergehenden Ansprüche, ferner umfassend einen Gleitstreifen (106S), der an der ersten Seitenwand angeordnet ist, und eine Schiebetür (106), die mit dem Gleitstreifen verbunden ist.

7. Schrank für medizinische Instrumente nach Anspruch 6, wobei die Schiebetür ein flexibles Paneel (106P) umfasst, das mit dem Gleitstreifen verbunden ist.

8. Schrank für medizinische Instrumente nach Anspruch 6 oder Anspruch 7, wobei die Schiebetür Griffe (106H) umfasst, die an dem flexiblen Paneel angebracht sind.

9. Schrank für medizinische Instrumente nach einem der Ansprüche 6 bis 8, wobei die Schiebetür keine Fugen aufweist.

## Revendications

1. Armoire d'instruments médicaux (100) comprenant :
une première paroi latérale (101),
une paroi arrière (103) raccordée à la première paroi latérale, et
un ensemble de support réglable (200) disposé sur la première paroi latérale ou la paroi arrière,
l'ensemble de support réglable comprenant :
un ensemble à coulisse (204), **caractérisé en ce que** l'ensemble de support réglable comprend en outre :
un boîtier (201, 202) qui est raccordé à l'ensemble à coulisse,
une fixation (203) mobile par rapport au boîtier et raccordée à l'ensemble à coulisse,
une courroie (205) raccordée à l'ensemble de fixation, et
au moins l'un d'un contrepoids (206) et d'un ressort de force constante (210) raccordé à la courroie.

2. Armoire d'instruments médicaux selon la revendication 1, dans lequel l'ensemble de fixation est au moins en partie revêtu d'un matériau élastomère.

3. Armoire d'instruments médicaux selon la revendication 1 ou la revendication 2, dans lequel l'ensemble à coulisse est un ensemble à coulisse à roulement à billes intégré.

4. Armoire d'instruments chirurgicaux selon l'une quelconque des revendications précédentes, comprenant en outre au moins l'une d'une poulie supérieure (207) et d'une poulie inférieure (208) disposées sur le boîtier.

5. Armoire d'instruments médicaux selon la revendication 4, dans laquelle la courroie vient en contact avec la au moins une de la poulie supérieure et de la poulie inférieure.

6. Armoire d'instruments médicaux selon l'une quelconque des revendications précédentes, comprenant en outre une bande glissante (106S) disposée sur la première paroi latérale, et une porte coulissante (106) raccordée à la bande glissante.

7. Armoire d'instruments médicaux selon la revendication 6, dans laquelle la porte coulissante comprend un panneau souple (106P) raccordé à la bande glissante.

8. Armoire d'instruments médicaux selon la revendication 6 ou la revendication 7, dans laquelle la porte coulissante comprend des poignées (106H) fixées au panneau souple.

9. Armoire d'instruments médicaux selon l'une quelconque des revendications 6 à 8, dans laquelle la porte coulissante n'a pas de jointures.
